(19) Europäisches Patentamt
European Patent Office
Office européen des brevets

(11) Veröffentlichungsnummer: **0 158 299**
**A2**

(12) **EUROPÄISCHE PATENTANMELDUNG**

(21) Anmeldenummer: 85104171.5

(22) Anmeldetag: 04.04.85

(51) Int. Cl.⁴: C 07 D 233/60
C 07 D 233/61, A 61 K 31/415

(30) Priorität: 09.04.84 DE 3413365

(43) Veröffentlichungstag der Anmeldung: 16.10.85 Patentblatt 85/42

(84) Benannte Vertragsstaaten: AT BE CH DE FR GB IT LI LU NL SE

(71) Anmelder: Merz & Co. GmbH & Co.
Eckenheimer Landstrasse 100-104
D-6000 Frankfurt/M. 1(DE)

(72) Erfinder: Scherm, Arthur, Dr.
Mittelstedter Weg 8
D-6380 Bad Homburg(DE)

(72) Erfinder: Peteri, Deszö, Dr.
Am Betzenberg 3
D-6751 Breunigweiler(DE)

(72) Erfinder: Deuschle, Eberhardt, Dr.
Günthersburgallee 14
D-6000 Frankfurt/M.(DE)

(72) Erfinder: Schatton, Wolfgang, Dr.
Dörnweg 11
D-6236 Eschborn(DE)

(74) Vertreter: Kinzebach, Werner, Dr.
Patentanwälte Reitstötter, Kinzebach und Partner
Postfach 780
D-8000 München 43(DE)

(54) **Substituierte Phenylethylderivate, Verfahren zu ihrer Herstellung und pharmazeutische Mittel.**

(57) Die Erfindung betrifft substituierte Phenylethylderivate, Verfahren zu ihrer Herstellung und pharmazeutische Mittel, die diese Verbindungen enthalten.

Die erfindungsgemäßen Verbindungen sind zur Bekämpfung von Pilzen und Bakterien brauchbar.

EP 0 158 299 A2

Croydon Printing Company Ltd

Die Erfindung betrifft substituierte Phenylethylderivate, Verfahren zu ihrer Herstellung und pharmazeutische Mittel, die diese Verbindungen enthalten. Die erfindungsgemäßen Verbindungen sind zur Bekämpfung von Pilzen und Bakterien brauchbar.

Es ist bekannt, daß Bakterien und insbesondere Pilze in immer stärkerem Maße auftreten. Obwohl beispielsweise eine Vielzahl an Mitteln zur Bekämpfung von Pilzen zur Verfügung steht, ist es häufig schwierig, Pilze wirksam zu behandeln, weil viele Mittel nur ganz spezifisch wirken und häufig Resistenzerscheinungen zu beobachten sind. Trotz der vielen bekannten Mittel besteht deshalb ein permanenter Bedarf an neuen Mitteln, die zur wirksamen Bekämpfung von Bakterien- und Pilzinfektionen brauchbar sind.

Aufgabe der Erfindung war es deshalb, neue, zur Bekämpfung von Bakterien und Pilzen wirksame Verbindungen, sowie Verfahren zur Herstellung dieser Verbindungen und pharmazeutische Mittel, welche diese Verbindungen enthalten, zu schaffen.

Diese Aufgabe wird gelöst durch Phenylethylderivate der allgemeinen Formel (I)

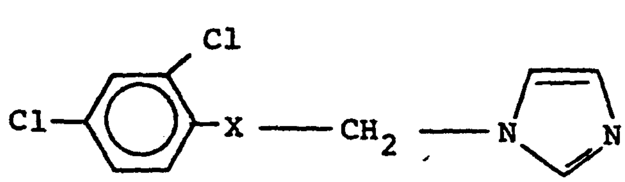

(I)

worin X für

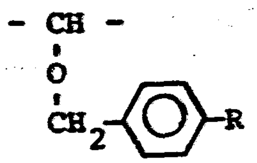

oder

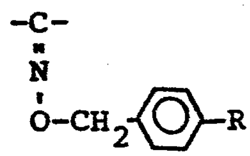

steht,

wobei R die 2,2-Dichlor-1,1-difluorethoxygruppe oder die 2-Chlor-1,1,2-trifluorethoxygruppe oder die 2,2-Dichlorcyclopropylgruppe bedeutet und deren pharmazeutisch verträglichen Säureadditionssalze.

Wenn X in den erfindungsgemäßen Verbindungen der allgemeinen Formel (I) für die Ethergruppierung steht, liegt ein asymetrisches Kohlenstoffatom vor. Wenn dagegen X die Oximethergruppierung bedeutet, können die erfindungsgemäßen Verbindungen in Form von E- bzw. Z-Isomeren auftreten. Die jeweiligen stereochemischen Isomerengemische können nach bekannten Verfahren aufgetrennt werden, die einzelnen Isomeren können jedoch auch stereospezifisch synthetisiert werden. Die Erfindung umfaßt auch die jeweiligen Stereoisomeren der erfindungsgemäßen Verbindungen.

Brauchbare pharmazeutisch verträgliche Säureadditionssalze erhält man durch Umsetzung mit anorganischen Säuren, wie Halogenwasserstoffsäuren, insbesonder Chlorwasserstoffsäure, Salpetersäure, Schwefelsäure, Phosphorsäure und dergleichen, und organischen Säuren, wie Essigsäure, Zitronensäure, Weinsäure, Milchsäure, Apfelsäure und dergleichen.

Das Verfahren zur Herstellung der erfindungsgemäßen Verbindungen ist dadurch gekennzeichnet, daß man eine Verbindung der allgemeinen Formel (II)

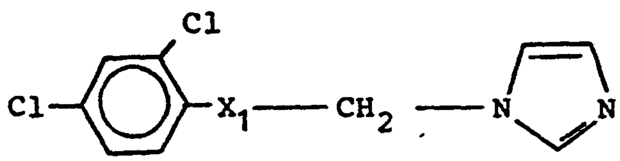

(II)

worin

$X_1$ für 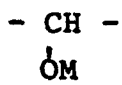 oder 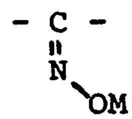

steht, wobei M für ein Wasserstoffatom oder ein Alkalimetall steht, mit einer Verbindung der allgemeinen Formel (III)

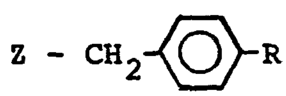

(III)

worin Z ein Halogenatom bedeutet und R die in Anspruch 1 angegebene Bedeutung besitzt, gegebenenfalls in Gegenwart einer Base und in einem geeigneten Lösungsmittel umsetzt, und gegebenenfalls die so erhaltenen Verbindungen der allgemeinen Formel (I) in pharmazeutisch verträgliche Säureadditionssalze überführt.

Für das erfindungsgemäße Verfahren brauchbare Basen sind dem Fachmann bekannt. Bevorzugte Basen sind Hydride, insbesondere Natriumhydrid.

Geeignete Lösungsmittel für diese Reaktionen sind reaktionsinerte organische Lösungsmittel, wie aromatische Kohlenwasserstoffe, z.B. Benzol, Toluol, Xylol und dergleichen; Alkylformamide, z.B. Dimethylformamid; Ether, z.B. Diethylether, Tetrahydrofuran, Dioxan und dergleichen; Aceton, Dichlormethan und Hexamethylphosphorsäuretriamid.

Die Reaktionstemperatur ist nicht entscheidend, vorteilhafterweise arbeitet man jedoch zur Beschleunigung der Reaktion bei höheren Temperaturen, die bis zum Siedepunkt des Lösungsmittels reichen können.

Das erfindungsgemäße Verfahren läßt sich insbesondere auch unter den Bedingungen der Phasen-Transfer-Katalyse durchführen.

Die Ausgangsverbindungen der allgemeinen Formel (II) werden durch Reduktion bzw. Oximierung der entsprechenden Ethanone erhalten, wie beispielsweise in J. Med. Chem. 1969, (12), 781 und Arzneim.-Forsch./Drug Res. 29(II), (10), 1510, (1979) beschrieben.

Die Benzylhalogenide der allgemeinen Formel (III) können nach bekannten Verfahren hergestellt werden, beispielsweise durch Umsetzung eines geeignet substituierten Toluols mit N-Bromsuccinimid.

1

Die erfindungsgemäßen Verbindungen besitzen ein breites fungizides und bakterizides Wirkungsspektrum und sind deshalb zur Bekämpfung von Pilzen und Bakterien brauchbar. Gegenüber gängigen pathogenen Pilzstämmen besitzen die erfindungsgemäßen Verbindungen minimale Hemmkonzentrationen im Bereich von 0,05 - 12,5 µg/ml.

Die erfindungsgemäßen Verbindungen werden im allgemeinen in Form von pharmazeutischen Mitteln verabreicht, die wenigstens eine der erfindungsgemäßen Verbindungen enthalten und gegebenenfalls in Kombination mit üblichen pharmazeutischen Trägern und/oder Hilfsstoffen formuliert sind.

Die nachfolgenden Beispiele erläutern die Erfindung.

<u>Beispiel 1</u>

<u>4-(2,2-Dichlor-1,1-difluorethoxy)benzylbromid:</u>

48,21 g (0,2 Mol) 4-(2,2-Dichlor-1,1-difluorethoxy)toluol, 39,16 g (0,22 Mol) N-Bromsuccinimid und 0,5 g 2,2'-Azoisobuttersäuredinitril werden in 200 $cm^3$ Tetrachlorkohlenstoff zum Sieden erhitzt und solange am Rückfluß erhitzt, bis die Reaktion beendet ist. Nach dem Abkühlen wird das Succinimid abfiltriert und das Lösungsmittel unter vermindertem Druck abdestilliert. Der Rückstand wird aus Hexan umkristallisiert und liefert 57 g 4-(2,2-Dichlor-1,1-difluorethoxy)-benzylbromid vom Schmelzpunkt 72°C.

Auf analoge Weise erhält man:

4-(2-Chlor-1,1,2-trifluorethoxy)benzylbromid

4-(2,2-Dichlorcyclopropyl)benzylbromid

Die hierzu als Ausgangsprodukte benötigten Toluole können auf folgende Weise erhalten werden:

Beispiel 2

4-(2,2-Dichlor-1,1-difluorethoxy)toluol aus 4-Methylphenol und 1,1-Difluor-1,2,2-trichlorethan:

21,6 g (0,2 Mol) 4-Methylphenol und 11,22 g (0,2 Mol) gepulvertes Kaliumhydroxid werden in 200 $cm^3$ Aceton 20 Minuten bei Raumtemperatur gerührt. Dann werden 33,9 g (0,2 Mol) 1,1-Difluor-1,2,2-trichlorethan zugegeben und das Gemisch 4 Stunden am Rückfluß erhitzt. Nach dem Abkühlen wird filtriert und das Lösungsmittel unter vermindertem Druck abdestilliert. Der Rückstand wird in Diethylether aufgenommen und mit verdünnter Natronlauge phenolfrei gewaschen. Dann wird 2 mal mit Wasser gewaschen und das Lösungsmittel unter vermindertem Druck abdestilliert. Der Rückstand wird im Vakuum destilliert und liefert 39,6 g 4-(2,2-Dichlor-1,1-difluorethoxy)toluol vom Siedepunkt 80°C/0,15 Torr.

Beispiel 3

4-(2,2-Dichlor-1,1-difluorethoxy)toluol aus 4-Methylphenol und 1,1-Dichlor-2,2-difluorethen

10,8 g (0,1 Mol) 4-Methylphenol in 100 $cm^3$ trockenem Tetrahydrofuran werden mit 0,24 g (0,01 Mol) Natriumhydrid versetzt. Nach Beendigung der Wasserstoffentwicklung werden 10 g (0,075 Mol) 1,1-Dichlor-2,2-difluorethen eingeleitet und das Gemisch 2 Stunden auf 60°C erwärmt. Dann wird, wie in Beispiel 2 beschrieben, aufgearbeitet. Auf diese Weise erhält man 15,5 g 4-(2,2-Dichlor-1,1-difluorethoxy)toluol.

Auf analoge Weise, jedoch im Autoklaven anstelle eines offenen Reaktionsgefäßes und unter Einsatz von trockenem Toluol als Lösungsmittel erhält man bei Einsatz des 1-Chlor-1,1,2-trifluorethens anstelle des 1,1-Dichlor-2,2-difluorethens das 4-(2-Chlor-1,1,2-trifluorethoxy)-toluol.

Beispiel 4

4-(2,2-Dichlorcyclopropyl)toluol

Zu 50 g (0,42 Mol) 4-Methylstyrol und 0,96 g (4,2 mMol) Benzyltrimethylammoniumchlorid in 150 $cm^3$ Chloroform werden bei 0°C unter starkem Rühren 75 g (1,88 Mol) Natriumhydroxid als 50%-ige wässrige Lösung zugetropft. Es wird 15 Minunten bei 0°C, 2 Stunden bei Raumtemperatur und 1,5 Stunden bei 50°C nachgerührt. Die organische

1

Phase wird abgetrennt und nach Trocknen über Magnesiumsulfat abdestilliert. Der Rückstand wird im Vakuum destilliert und liefert 74,2 g 4-(2,2-Dichlorcyclopropyl)-toluol vom Siedepunkt 120 - 122°C/14 Torr.

## Beispiel 5

1-(2,4-Dichlorphenyl)-2-(1H-imidazol-1-yl)-O-(4-(2,2-dichlor-1,1-difluorethoxy)benzyl)ethanonoxim-nitrat (I):

Zu 100 $cm^3$ trockenem Tetrahydrofuran werden nacheinander 0,48 g (0,02 Mol) Natriumhydrid, 5,40 g (0,02 Mol) 1-(2,4-Dichlorphenyl)-2-(1H-imidazol-1-yl)ethanonoxim und 6,4 g (0,02 Mol) 4-(2,2-Dichlor-1,1-difluorethoxy)benzylbromid gegeben und bei Raumtemperatur 6 Stunden gerührt. Danach wird filtriert und das Lösungsmittel unter vermindertem Druck abdestilliert. Der Rückstand wird in Isopropanol aufgenommen und mit konzentrierter Salpetersäure im Überschuß versetzt. Die bei Zugabe von Wasser ausfallende zähe Masse wird durch Dekantieren von der wässrigen Phase befreit und aus Toluol umkristallisiert. Man erhält 8,2 g 1-(2,4-Dichlorphenyl)-2-(1H-imidazol-1-yl)-O-(4-(2,2-dichlor-1,1-difluorethoxy)benzyl)ethanonoxim-nitrat.

Schmp.: 134°C

Summenformel: $C_{20}H_{16}Cl_4F_2N_4O_5$

Molekulargewicht: 572,18

| Elementaranalyse: | C | H | Cl | F | N |
|---|---|---|---|---|---|
| berechnet: | 41,98 | 2,82 | 24,78 | 6,64 | 9,79 |
| gefunden: | 42,14 | 2,63 | | | 9,91 |

Weitere Beispiele der auf analoge Weise erhaltenen Phenethylimidazolderivate sind:

1-(2,4-Dichlorphenyl)-2-(1H-imidazol-1-yl)-O-(4-(2,2-dichlorcyclopropyl)benzyl)ethanonoxim-nitrat (II):

Schmp.: 142°C
Summenformel: $C_{21}H_{18}Cl_4N_4O_4$
Molekulargewicht: 532,21

| Elementaranalyse: | C | H | Cl | N |
|---|---|---|---|---|
| berechnet: | 47,39 | 3,41 | 26,65 | 10,53 |
| gefunden: | 47,43 | 3,45 | 26,61 | 10,38 |

1-(2,4-Dichlorphenyl)-1-(4-(2,2-dichlorcyclopropyl)benzyloxy)-2-(1H-imidazol-1-yl)ethan-nitrat (III):

Schmp.: 153°C
Summenformel: $C_{21}H_{19}Cl_4N_3O_4$
Molekulargewicht: 519,22

| Elementaranalyse: | C | H | Cl | N |
|---|---|---|---|---|
| berechnet: | 48,58 | 3,69 | 27,31 | 8,09 |
| gefunden: | 48,69 | 3,68 | 27,20 | 7,99 |

1-(2,4-Dichlorphenyl)-1-(4-(2,2-dichlor-1,1-difluorethoxy)benzyloxy)-2-(1H-imidazol-1-yl)ethan-hydrochlorid (IV):

Schmp.: 232°C
Summenformel: $C_{20}H_{17}Cl_5F_2N_2O_2$
Molekulargewicht: 532,63

| Elementaranalyse: | C | H | Cl | N |
|---|---|---|---|---|
| berechnet: | 45,10 | 3,22 | 33,28 | 5,26 |
| gefunden: | 45,15 | 3,40 | 33,36 | 5,17 |

1-(2,4-Dichlorphenyl)-1-(4-(2,2-dichlor-1,1-difluorethoxy)benzyloxy)-2-(1H-imidazol-1-yl)ethan-nitrat (V):

Schmp.: 141°C
Summenformel: $C_{20}H_{17}Cl_4F_2N_3O_5$
Molekulargewicht: 558,18

| Elementaranalyse: | C | H | Cl | F | N |
|---|---|---|---|---|---|
| berechnet: | 42,96 | 3,06 | 25,36 | 6,80 | 7,51 |
| gefunden: | 43,13 | 3,15 | 25,19 | 6,91 | 7,41 |

1-(2,4-Dichlorphenyl)-1-(4-(2-chlor-1,1,2-trifluorethoxy)benzyloxy)-2-(1H-imidazol-1-yl)ethan-nitrat (VI):

Schmp.: 130°C
Summenformel: $C_{20}H_{17}Cl_3F_3N_3O_5$
Molekulargewicht: 542,73

| Elementaranalyse: | C | H | Cl | F | N |
|---|---|---|---|---|---|
| berechnet: | 44,26 | 3,16 | 19,60 | 10,50 | 7,74 |
| gefunden: | 44,39 | 3,18 | 19,47 | 10,42 | 7,60 |

Die erfindungsgemäßen Verbindungen wurden im Reihenverdünnungstest auf ihre fungistatische Wirksamkeit gegen

verschiedene Pilzspezies und gegen verschiedene Bakterienstämme geprüft. Die so ermittelten minimalen Hemmkonzentrationen (MHK) sind in den folgenden Tabellen wiedergegeben:

**Tabelle 1:** Minimale Hemmkonzentrationen in $\mu$g/ml

| | Pilzspezies | | | | | | | |
|---|---|---|---|---|---|---|---|---|
| Verbind. | C.a. | C.t. | A.c. | A.f. | T.r. | T.m. | M.c. | S.s. |
| I | 0,1 | 0,8 | 1,6 | 12,5 | 6,25 | 6,25 | 3,1 | 6,25 |
| II | 3,1 | 25 | 6,2 | 25 | 6,2 | 12,5 | 6,2 | 1,6 |
| III | 0,05 | 3,1 | 1,6 | 1,6 | 0,8 | 0,8 | 0,8 | 0,2 |
| IV | 0,1 | 0,1 | 3,1 | 3,1 | 0,1 | 0,1 | 0,1 | 3,1 |
| VI | 0,05 | 1,6 | 0,8 | 1,6 | 0,8 | 0,8 | 0,2 | 0,1 |
| Ref. | 1,6 | 6,2 | 25 | 50 | 6,2 | 6,2 | 12,5 | >50 |

| | | |
|---|---|---|
| Ref. | = | Referenz: Nystatin |
| C.a. | = | Candida albicans |
| C.t. | = | Candida tropicalis |
| A.c. | = | Absidia corymbifera |
| A.f. | = | Aspergillus fumigatus |
| T.r. | = | Trichophyton rubrum |
| T.m. | = | Trichophyton mentagrophytes |
| M.c. | = | Microsporum canis |
| S.s. | = | Sporotrichum schenkii |

Tabelle 2: Minimale Hemmkonzentrationen in µg/ml

| | Bakterienstämme | | |
|---|---|---|---|
| Verbind. | S.a. | S.p. | S.f. |
| I | 6,25 | 0,8 | 12,5 |
| IV | 3,1 | $<$0,2 | 1,6 |

| | | |
|---|---|---|
| S.a. | = | Staphylococcus aureus |
| S.p. | = | Streptococcus pyogenes |
| S.f. | = | Streptococcus faecalis |

M/26 025

# Patentansprüche

1. Substituierte Phenylethylderivate der allgemeinen Formel (I)

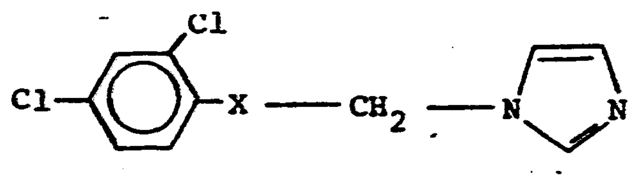

(I)

worin X für

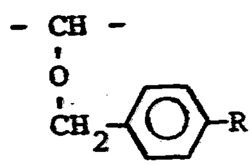

oder

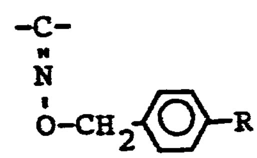

steht,

wobei R die 2,2-Dichlor-1,1-difluorethoxygruppe, die 2-Chlor-1,1,2-trifluorethoxygruppe oder die 2,2-Dichlorcyclopropylgruppe bedeutet und deren pharmazeutisch verträglichen Säureadditionssalze.

2. Verfahren zur Herstellung der Verbindungen nach Anspruch 1, dadurch gekennzeichnet, daß man eine Verbindung der allgemeinen Formel (II)

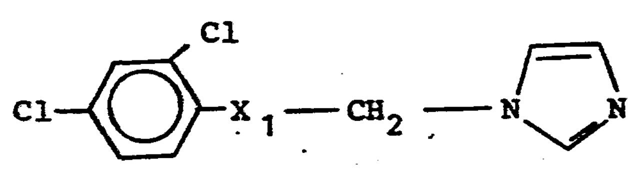

worin

$X_1$ für 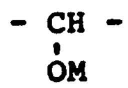 oder 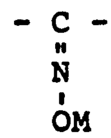

steht,
wobei M für ein Wasserstoffatom oder ein Alkalimetall steht, mit einer Verbindung der allgemeinen Formel (III)

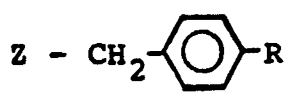

worin Z ein Halogenatom bedeutet und R die in Anspruch 1 angegebenen Bedeutungen besitzt, gegebenenfalls in Gegenwart einer Base und in einem geeigneten Lösungsmittel umsetzt und gegebenenfalls die so erhaltenen Verbindungen der allgemeinen Formel (I) in pharmazeutisch verträgliche Säureadditionssalze überführt.

3. Pharmazeutische Mittel, enthaltend wenigstens eine der Verbindungen nach Anspruch 1, gegebenenfalls in Kombination mit üblichen pharmazeutischen Trägern und/oder Hilfsmitteln.

4. Verwendung der Verbindungen nach Anspruch 1 zur Herstellung eines pharmazeutischen Mittels zur Bekämpfung von Pilzen und Bakterien.